# EUROPEAN PATENT APPLICATION

(11) **EP 3 446 754 A1**
(43) Date of publication of application: **27.02.2019**
(21) Application number: 17187614.7
(22) Date of filing: 24.08.2017
(51) Int. Cl.: A62B 7/10, A62B 18/00, A62B 18/04, H02P 6/00, A61M 16/00

(54) **A MASK-BASED BREATHING APPARATUS AND CONTROL METHOD**

(71) Applicant: Koninklijke Philips N.V., 5656 AE Eindhoven (NL)
(72) Inventor: VAN DER SLUIS, Paul, 5656 AE Eindhoven (NL)
(74) Representative: de Haan, Poul Erik

(57) **Abstract**

The invention provides a breathing assistance mask having a fan which comprises a brushless DC motor. A start-up circuit is provided for supplying a time varying drive voltage as the supply voltage for the stator electromagnet of the motor, with a high voltage start-up drive voltage followed by a steady state drive voltage. This high voltage start-up signal ("high" relative to the steady state drive voltage) enables the fan to reach its operating speed with a reduced time delay.

## Description

### FIELD OF THE INVENTION

This invention relates to a breathing apparatus in the form of a mask, for providing filtered air to the wearer of the breathing apparatus, with the flow assisted by a fan.

### BACKGROUND OF THE INVENTION

The World Health Organization (WHO) estimates that 4 million people die from air pollution every year. Part of this problem is the outdoor air quality in cities. The worst in class are Indian cities like Delhi that have an annual pollution level more than 10 times the recommended level. Well known is Beijing with an annual average 8.5 times the recommended safe levels. However, even in European cities like London, Paris and Berlin, the levels are higher than recommended by the WHO.

Since this problem will not improve significantly on a short time scale, the only way to deal with this problem is to wear a mask which provides cleaner air by filtration. To improve comfort and effectiveness one or two fans can be added to the mask. These fans are switched on during use and are typically used at a constant voltage. For efficiency and longevity reasons these are normally electrically commutated brushless DC fans.

The benefit to the wearer of using a powered mask is that the lungs are relieved of the slight strain caused by inhalation against the resistance of the filters in a conventional non-powered mask. A non-powered mask also can give discomfort to the face due to high temperature and humidity build-up inside the mask. A powered mask with an integrated fan reduces this discomfort.

Furthermore, in a conventional non-powered mask, inhalation also causes a slight negative pressure within the mask which leads to leakage of the contaminants into the mask, which leakage could prove dangerous if these are toxic substances. A powered mask delivers a steady stream of air to the face and may for example provide a slight positive pressure, which may be determined by the resistance of an exhale valve, to ensure that any leakage is outward rather than inward.

There are several configurations possible for adding a fan. There may be a fan at the inlet or outlet or both, with or without the filter in series and with or without a check valve in series. All of these configurations have advantages and disadvantages in terms of cost and improved comfort.

There are several advantages if the fan operation (or just speed) is regulated. This control can be used to improve comfort by more appropriate ventilation during the inhalation and exhalation sequence or it can be used to improve the electrical efficiency. Fan-operated masks are battery-operated devices, so that it is desirable to reduce power consumption to a minimum as well as keeping the cost to a minimum.

For example, GB 2032284 discloses a respirator in which the pressure inside a mask is measured by a pressure sensor and the fan speed is varied in dependence on the sensor measurements.

Significant improvements in comfort and freedom in configuration choice can be achieved if the fan or fans can be switched on and/or off quickly. However, fans are generally designed for continuous operation and thus are not optimized for quick start and stop functions.

There remains a need for a fan control approach which enables fast start-up or stopping of a fan, so that the timing of fan operation can be controlled accurately and so that the fan function is ready with minimum initial delay.

### SUMMARY OF THE INVENTION

The invention is defined by the claims.

According to examples in accordance with an aspect of the invention, there is provided a mask comprising:
an air chamber;
a filter;
a fan for drawing air from outside the air chamber into the air chamber and/or drawing air from inside the air chamber to the outside, wherein the fan comprises a brushless DC motor which comprises:
   a rotor having a set of rotor magnets;
   a stator electromagnet arrangement;
   a magnetic field detector for detecting a rotational position of the rotor; and
   a supply circuit for supplying the stator electromagnet with a timing dependent on the output of the magnetic field detector; and
a start-up circuit adapted to provide a time varying drive voltage as the supply voltage for the stator electromagnet arrangement in the form of a high voltage start-up drive voltage followed by a steady state drive voltage.

The stator electromagnet arrangement comprises a set of one or more stator magnets.

This mask design enables the fan to be started with increased speed by increasing the stator electromagnet drive voltage during an initial start-up time. In this way, efficiency savings are possible by using the fan only when needed, as it can quickly be turned on and off. Thus, timed fan operation, for example synchronized with the breathing cycle of the user, is more easily implemented.

The term "high voltage" is intended to mean a high voltage relative to the steady state drive voltage, i.e. higher than the steady state drive voltage. The steady state drive voltage is a normal operating voltage, i.e. one which can be maintained over time and meet the driving requirements of the fan.

The start-up circuit is for example adapted to apply the high voltage start-up drive voltage for a period of less than 0.25 s. Thus, it has only a short term overdrive function. The high voltage start-up drive voltage may even last for a period of less than 0.1 s. The start-up function may be used each time the fan is driven from a no rotation state to a normal rotation state.

There may also be a high speed braking function for rapid switching off of the fan, again by using a time varying drive voltage. This will involve applying different (negative) or differently timed (out of phase) voltages on the stator electromagnet arrangement.

The start-up circuit may be adapted to apply a high voltage start-up drive voltage which is at least two times the steady state drive voltage or at least three times the steady state drive voltage. This provides a short duration relatively high voltage drive pulse to deliver sufficient energy to increase the air flow and to increase the fan blade rotational speed.

The mask preferably includes a control circuit which is supplied with a constant supply voltage. The magnetic field detector may also be driven by (i.e. supplied by) a constant drive voltage. Thus, the overdrive is used only for the stator electromagnet coil, and the magnetic field detector and the control circuit do not need to be able to tolerate a fluctuating voltage source.

The mask may comprise a control circuit in the form of a motor controller integrated circuit and the supply circuit comprises a set of switches which is controlled by the motor controller integrated circuit. The integrated circuit may also be driven by a constant drive voltage.

Examples in accordance with another aspect of the invention provide a control method for operating a fan of a mask, wherein the mask comprises an air chamber, a filter and the fan for drawing air from outside the air chamber into the air chamber and/or drawing air from inside the air chamber to the outside, wherein the fan comprises a brushless DC motor which comprises a rotor having a set of rotor magnets, a stator electromagnet arrangement, a magnetic field detector for detecting a rotational position of the rotor and a supply circuit for supplying the stator electromagnet arrangement with a timing dependent on the output of the magnetic field detector,
wherein the method comprises:
when start-up of the fan is desired, providing a time varying drive voltage as the supply voltage for the stator electromagnet in the form of a high voltage start-up drive voltage followed by a steady state drive voltage.

This method enables a rapid start-up of a mask fan. The fan may be switched on and off rapidly to save power, and it may react quickly to increase user comfort.

The method may comprise applying the high voltage start-up drive voltage for a period of less than 0.25 s, for example for a period of less than 0.1 s, and the high voltage start-up drive voltage may be at least two times the steady state drive voltage, for example at least three times the steady state drive voltage.

The method may comprise driving the magnetic field detector by a constant drive voltage, and a motor controller integrated circuit may also be driven to control a set of switches which form the supply circuit.

The invention may be implemented at least in part in software.

### BRIEF DESCRIPTION OF THE DRAWINGS

Examples of the invention will now be described in detail with reference to the accompanying drawings, in which:
Figure 1 shows a face mask to which the invention may be applied;
Figure 2 shows schematically the brushless DC motor used in the fan;
Figure 3 shows an H-bridge circuit which functions as an inverter to generate an alternating voltage to the stator electromagnet coil from a DC supply;
Figure 4 shows an arrangement in accordance with an example of the invention;
Figure 5 shows one example of the voltage waveform applied by the start-up circuit;
Figure 6 shows an implementation using a control integrated circuit; and
Figure 7 shows schematic timing diagrams for a standard drive scheme and two drive schemes in accordance with examples of the invention.

### DETAILED DESCRIPTION OF THE EMBODIMENTS

The invention provides a breathing assistance mask having a fan which comprises a brushless DC motor. A start-up circuit is provided for supplying a time varying drive voltage as the supply voltage for the stator electromagnet arrangement of the motor, with a high voltage start-up drive voltage followed by a steady state drive voltage. This high voltage start-up voltage ("high" relative to the steady state drive voltage) enables the fan to reach its operating speed with a reduced time delay.

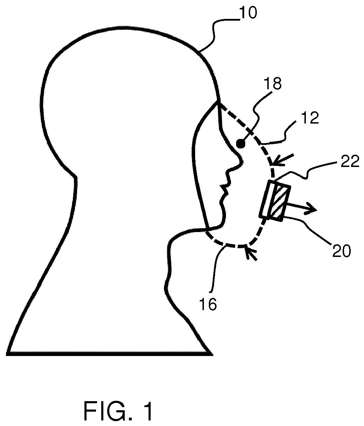

Figure 1 shows a subject 10 wearing a face mask 12 which covers the nose and mouth of the subject. The purpose of the mask is to filter air before it is breathed in the subject. For this purpose, in this example the mask body itself acts as an air filter 16. Air is drawn in to an air chamber 18 formed by the mask by inhalation. During inhalation, an outlet valve 22 such as a check valve is closed due (or just coinciding with) to the lower pressure in the air chamber 18.

When the subject breathes out, air is exhausted through the outlet valve 22. This valve is opened to enable easy exhalation, but is closed during inhalation. A fan 20 assists in the removal of air through the outlet valve 22. Preferably, more air is removed than exhaled so that additional air is supplied to the face. This increases comfort due to lowering relative humidity and cooling.

Between inhalation and exhalation, the fan operation increases comfort because fresh air is sucked into the air chamber thereby cooling that space.

During inhalation, by closing the valve, it is prevented that unfiltered air is drawn in. The timing of the outlet valve 22 is thus dependent on the breathing cycle of the subject. The outlet valve may be a simple passive check valve operated by the pressure difference across the filter 16. However, it may instead be an electronically controlled valve.

In a most basic implementation, the fan is on continuously. Clean air is in this way continuously supplied to the mask volume, either through the wall of the mask or through an inline filter.

However, during inhalation, the fan can optionally be switched off to save power, so that there is only fan assisted exhalation. This provides a mode of operation which relies on detecting the respiration cycle. For the fan assisted exhalation, power needs to be restored just before the exit valve opens again. This also makes sure that the next inhale-exhale cycle remains properly timed and sufficient pressure and flow are made available.

Around 30% power savings are easily achievable using this approach, resulting in prolonged battery life. Alternatively, the power to the fan can be increased by 30% for enhanced effectiveness.

This is only one example of possible configuration. There may a single fan which maintains a positive pressure inside the mask or there may be separate fans for inhalation and exhalation.

When used in a discontinuous mode, a fan (or fans) is (or are) able to track the breathing cycle of the user, to make breathing in the mask more comfortable. For example, an inlet fan present in a mask may rotate during inhalation and an outlet fan may rotate during exhalation. An inlet fan for example may draw air through a filter (rather than through the wall of the face mask which may then be gas impermeable) from outside the air chamber into the air chamber. An inlet fan may be positioned before or after such a filter. An outlet fan for example may draw air from inside the air chamber to the outside and may also only operate during exhalation.

The inlet fan or outlet fan may in this way be used to assist the breathing of the user. If both an inlet fan and an outlet fan are provided, the inhalation/exhalation cycle may then be fully assisted, with the inlet and outlet fans synchronized with the breathing cycle of the user.

When the fan operation is to be synchronized with the breathing cycle, a differential pressure sensor may be used to determine a difference in pressure between air outside the device and air inside the mask. This provides accurate monitoring of the breathing cycle of the user with a minimal time lag between the detected variation of differential pressure over time and the inhalation and exhalation timing of the user.

In all cases, there can be a significant time delay in bringing the fan rotation up to speed, either at initial turn on (for a continuous fan) or at each timing instant (for a fan with rotation synchronized to the breathing cycle).

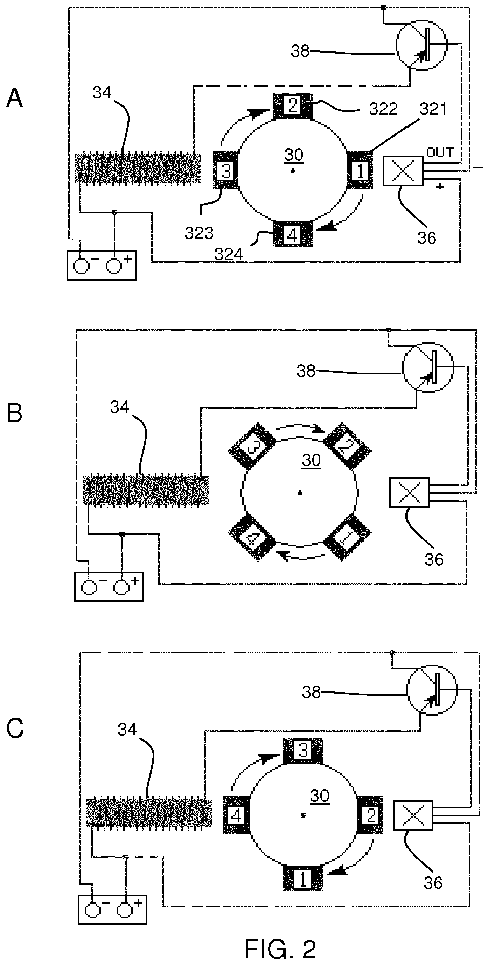

Figure 2 shows schematically the brushless DC motor used in the fan.

It comprises a rotor 30 having a set of rotor magnets 321, 322, 323, 324. A set of four rotor magnets is simply one example, and other number of rotor magnets may of course be used. A stator electromagnet 34 is used to apply a force to a rotor magnet in its proximity. There may be on stator magnet or a set of stator electromagnets. Multiple stator electromagnets for example have different phases. A magnetic field detector 36 such as a Hall sensor is used for detecting the proximity of a rotor magnet in order to generate a timing signal for controlling the operation of the stator electromagnet 34. In particular, in the simplified schematic shown, the signal from the magnetic field detector 36 couples to the base of a control power transistor 38 which in turn controls the supply of a drive voltage to the stator electromagnet 34. The electromagnet 34 is coupled between a high power supply and the low power supply terminal (e.g. ground) through the transistor 38.

For simplicity, Figure 2 shows a simple one phase design and it shows only a simple control transistor. In practice, there is a control integrated circuit, for example making sure the rotation direction is fixed.

Figure 2 shows three sequential rotational positions of the rotor.

In Figure 2A, the magnet 321 is close to the Hall sensor 36, so the sensor 36 sends a signal to the base of the power transistor 38. The transistor opens, and allows a large collector current to flow through the electromagnet 34 and through the transistor to the low power supply terminal. The electromagnet pushes magnet 323 away to induce clockwise rotation.

In Figure 2B the rotor 30 has spun away so that magnet 322 stops affecting the Hall sensor 36. Since the signal to the base of the power transistor has been removed, it is turned off. This disables the electromagnet 34.

In Figure 2C, the rotor continues to spin due to its inertia until magnet 322 moves into the working range of the Hall sensor 36. The Hall sensor sends a signal to the base of the transistor 38. The transistor opens, and allows a collector current to flow through the electromagnet. The electromagnet 34 pushes magnet 324 away. This process continues until the power is disconnected.

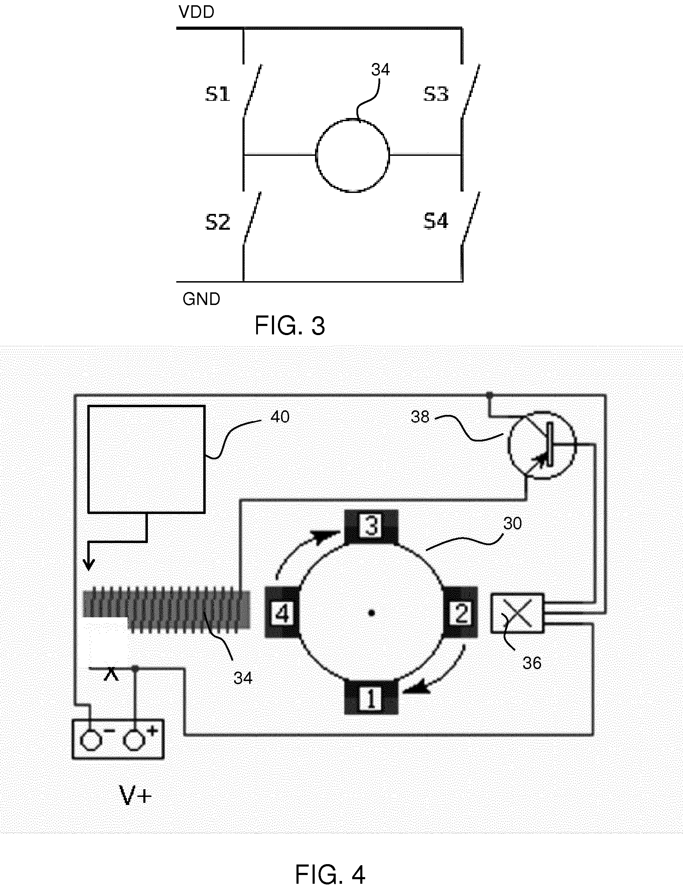

In practice, the control circuit is more complicated. For example, Figure 3 shows an H-bridge circuit which functions as an inverter to generate an alternating voltage to the stator electromagnet coil 34 from a DC supply VDD, GND. The inverter has a set of switches S1 to S4 to generate an alternating voltage across the coil 34. Thus, the signal transistor 38 in Figure 2 functions as one of the switches S1 to S4, and the switches are for example controlled by a controller integrated circuit. The H bridge circuit enables bidirectional control of the current through the electromagnet, so that phases of attraction and repulsion may be provided during rotation of the rotor. The controller IC receives the sensed signal from the magnetic field detector.

It takes time for the motor and hence the fan to reach its operational speed. Energy is required for the air to start moving (translational kinetic energy) and energy is required for the fan blade rotation (rotational kinetic energy). When constant power is supplied to the fan motor, that process may take considerable time.

In order to speed up the start-up process, the invention provides an arrangement by which more power is supplied to the motor during the start-up phase. For a significant and usable reduction in start-up time, the stator current may for example be increased by a factor of more than 2, for example more than 5 and for example more than 8. This large peak in current can be tolerated by the motor circuitry because the peak power allowed through the coils for a very short period of time is much higher than the continuous power that can be applied to the coil.

In the example shown in Figure 2, the stator electromagnet coil 34 is always connected to the same power supply (with voltage V+) as the Hall sensor 36 (and the control IC in a real implementation).

Figure 4 shows an arrangement in accordance with one example of the invention. In general terms, the transistor 38 is part of a supply circuit for supplying the stator electromagnet 34 with a timing dependent on the magnetic field detector 36. As shown above, the supply circuit may include multiple transistors (or other switches) to enable bidirectional control.

The circuit additionally comprises a start-up circuit 40 which is adapted to provide a time varying drive voltage to the stator electromagnet coil 34 in the form of a high voltage start-up drive voltage followed by a steady state drive voltage.

Thus, the stator electromagnet coil is no longer connected to the constant power supply V+ but is connected to a variable power supply. The magnetic field detector 36 and the controller IC (not shown in Figure 4) remain connected to the constant voltage supply.

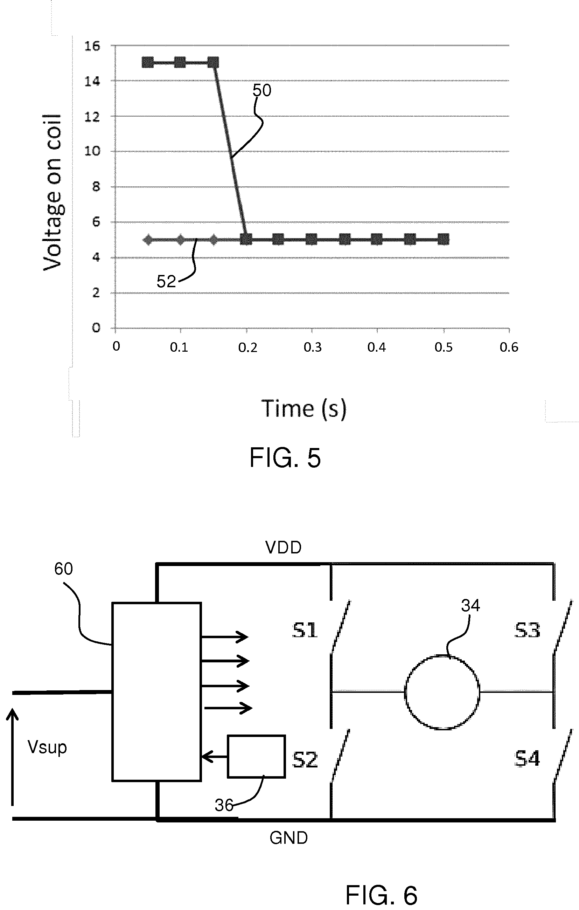

Figure 5 shows one example of the voltage waveform applied by the start-up circuit 40. Plot 50 is the voltage waveform, and plot 52 is the conventional constant voltage supply.

At initial fan start-up, a high voltage start-up drive voltage of 15V is applied for 0.15s before the steady state drive voltage of 5V is applied. Thus, the voltage is increased by a factor of 3 during the start-up phase. This results in a nearly 3 fold current increase, which decreases the start-up time by nearly a factor of 3. The precise length and height of the voltage peak depends on the fan motor and the requirements for the start-up time.

For applications where the fan is to be started up synchronized with inhalations or exhalations, the start-up period is sufficiently short that the fan speed may be reached after only a small fraction of the time of the inhalation or exhalation duration. By way of example the start-up period, during which the start-up circuit applies the high voltage start-up drive voltage, is preferably less than 0.25 s, more preferably less than 0.1 s.

The high voltage start-up drive voltage is preferably at least two times the steady state drive voltage, more preferably at least three times the steady state drive voltage. It may even be as high as 5 to 10 times the steady state drive voltage.

Figure 6 shows an implementation using a control integrated circuit 60, which receives the signal from the Hall sensor 36 (and provides a stable static power supply to the Hall sensor) and generates the control signals for the switches S1 to S4. It also generates the variable voltage supply which is coupled to the stator electromagnet arrangement 34 by the switches S1 to S4. The control circuit is however supplied with a constant voltage.

The approach of the invention allows the fan rotation to get up to speed more quickly as explained above. In addition, if the high voltage pulse is applied for longer than required for the fan to reach its desired end speed, the fan speed may be brought to a higher level than the nominal fan speed for a short period of time. That short period of extra high rotation speed may then be used to bring the air flow faster up to its desired level. Apart from the rotational inertia of the fan rotor, also the translational inertia of the moving air mass has to be overcome before the fan is providing the desired air flow rate.

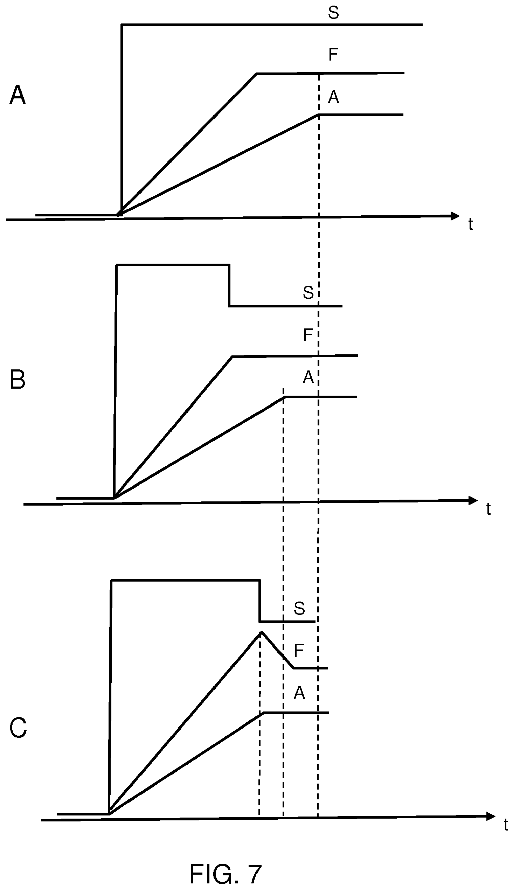

This approach is illustrated schematically in Figure 7. The stator electromagnet arrangement voltage is shown as "S", the fan rotation speed is shown as "F" and the air flow rate is shown as "A".

Figure 7A shows a conventional drive scheme.

Figure 7B shows a first example of drive scheme in accordance with the invention, providing a higher voltage until the fan speed has reached its desired level. The time to reach the desired air flow rate is reduced.

Figure 7C shows a second example of drive scheme in accordance with the invention, providing a higher voltage until the air flow rate has reached its desired level as explained above. The time to reach this desired air flow rate is reduced further (but by a relatively small amount as explained below).

The duration of the higher voltage pulse, including the additional duration to overcome the air inertia, depends on the fan configuration. The masses which are accelerated are of comparable order, based on the assumption that the weight of the fan rotor is comparable to the weight of the air that has to be set in motion. However, the fan rotor inertia is dominant because of the much greater fan speed (e.g. a fan rotating at 5000 rpm) compared to the linear air flow speed (e.g. 0.1 m/s).

Thus, the kinetic energy required to be delivered to the rotating fan rotor is dominant, and only a small additional time period (if any at all) is needed to reach the desired air flow speed.

Additional protection circuits may be required to control back EMF that will be stronger because of the higher voltages.

Fast stopping of the fan if required can be performed by similar means by using a short duration higher drive voltage, with a suitable phase of the voltage supplied to the coil. Stopping the fan may be achieved by active braking using the stator electromagnet coil with suitable current direction and timing.

Braking may be achieved with timing change, for example pushing on a rotor magnet before it reaches the stator electromagnet coil position, or by switching the stator between attraction and repulsion, so repelling an approaching rotor coil and attracting a rotor coil which is moving away. These measures may be combined. Indeed, the normal fan driving function may use repulsion as well as attraction or just one of these. The bidirectional control enabled by the H-bridge allows for control of attraction and repulsion.

Figure 5 shows a supply voltage for the stator which has only two levels. Of course, more complicated supply voltage waveforms may be used. The start-up voltage waveform may be applied without any modification to the timing sequence implemented by the controller IC, so that the system is simple to implement.

In the start-up phase, the rotor will be rotating at a lower speed. The control pulses will have timing which depends on this rotor speed, but this is part of the normal feedback control implemented by the Hall sensor and controller circuit.

The stator supply voltage is not controlled as part of the normal control of the speed of the fan. Instead, fan speed control is implemented based on the switch signals. Thus, the control of the stator supply is used only to implement the rapid start-up function.

As discussed above, embodiments make use of a controller, which can be implemented in numerous ways, with software and/or hardware, to perform the various functions required. A processor is one example of a controller which employs one or more microprocessors that may be programmed using software (e.g., microcode) to perform the required functions. A controller may however be implemented with or without employing a processor, and also may be implemented as a combination of dedicated hardware to perform some functions and a processor (e.g., one or more programmed microprocessors and associated circuitry) to perform other functions.

Examples of controller components that may be employed in various embodiments of the present disclosure include, but are not limited to, conventional microprocessors, application specific integrated circuits (ASICs), and field-programmable gate arrays (FPGAs).

In various implementations, a processor or controller may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor or controller.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

## Claims

1. A mask comprising:
an air chamber (18);
a filter (16);
a fan (20) for drawing air from outside the air chamber (18) into the air chamber and/or drawing air from inside the air chamber to the outside, wherein the fan comprises a brushless DC motor which comprises:
a rotor (30) having a set of rotor magnets (321, 322, 323, 324);
a stator electromagnet arrangement (34);
a magnetic field detector (36) for detecting a rotational position of the rotor; and
a supply circuit (36, S1-S4) for supplying the stator electromagnet with a timing dependent on the output of the magnetic field detector; and
a start-up circuit (40) adapted to provide a time varying drive voltage as the supply voltage for the stator electromagnet arrangement in the form of a high voltage start-up drive voltage followed by a steady state drive voltage.

2. A mask as claimed in claim 1, wherein the start-up circuit (40) is adapted to apply the high voltage start-up drive voltage for a period of less than **0.25** s, for example less than **0.1** s.

3. A mask as claimed in any preceding claim, wherein the start-up circuit (40) is adapted to apply a high voltage start-up drive voltage which is at least two times the steady state drive voltage.

4. A mask as claimed in claim 3, wherein the start-up circuit (40) is adapted to apply a high voltage start-up drive voltage which is at least three times the steady state drive voltage.

5. A mask as claimed in any preceding claim, comprising a control circuit (60) which is supplied with a constant supply voltage.

6. A mask as claimed in any preceding claim, wherein the magnetic field detector (36) is supplied with a constant supply voltage.

7. A mask as claimed in any preceding claim, comprising a control circuit in the form of a motor controller integrated circuit (60) and the supply circuit comprises a set of switches (S1-S4) which is controlled by the motor controller integrated circuit.

8. A control method for operating a fan of a mask, wherein the mask comprises an air chamber (18), a filter (16) and the fan (20) for drawing air from outside the air chamber (18) into the air chamber and/or drawing air from inside the air chamber to the outside, wherein the fan comprises a brushless DC motor which comprises a rotor (30) having a set of rotor magnets (321, 322, 323, 324), a stator electromagnet arrangement (34), a magnetic field detector (36) for detecting a rotational position of the rotor and a supply circuit (38, S1-S4) for supplying the stator electromagnet arrangement with a timing dependent on the output of the magnetic field detector,
wherein the method comprises:
when start-up of the fan is desired, providing a time varying drive voltage as the supply voltage for the stator electromagnet in the form of a high voltage start-up drive voltage followed by a steady state drive voltage.

9. A method as claimed in claim 8, comprising applying the high voltage start-up drive voltage for a period of less than **0.25** s.

10. A method as claimed in claim 9, comprising applying the high voltage start-up drive voltage for a period of less than **0.1** s.

11. A method as claimed in any one of claims 8 to 10, comprising applying a high voltage start-up drive voltage which is at least two times the steady state drive voltage.

12. A method as claimed in claim 11, comprising applying a high voltage start-up drive voltage which is at least three times the steady state drive voltage.

13. A method as claimed in any one of claims 8 to 12, comprising driving the magnetic field detector by a constant drive voltage.

14. A method as claimed in any one of claims 8 to 13, comprising driving a motor controller integrated circuit to control a set of switches which form the supply circuit.

15. A computer program comprising computer program code means which is adapted, when said program is run on a computer, to perform the method of any one of claims 8 to 14.
